# EUROPÄISCHE PATENTSCHRIFT

(11) **EP 0 820 991 B1**
(45) Veröffentlichungstag und Bekanntmachung des Hinweises auf die Patenterteilung: **29.08.2007**
(21) Anmeldenummer: 97112198.3
(22) Anmeldetag: 17.07.1997
(51) Int. Cl.: C07D 239/16, C07D 233/48, C07C 279/22, C07C 279/20, A61K 31/415, A61K 31/505

(54) **Cycloalkyl-Derivate als Inhibitoren der Knochenresorption und Vitronectinrezeptor-Antagonisten**
Cycloalkyl derivatives as bone resorption inhibitors and vitronectin receptor antagonists
Dérivés cycloalkyle comme inhibiteurs de la résorption osseuse et comme antagonistes du récepteur de la vitronectine

(30) Priorität: 24.07.1996 DE 19629816
(43) Veröffentlichungstag der Anmeldung: 28.01.1998
(73) Patentinhaber: HOECHST AKTIENGESELLSCHAFT, 65929 Frankfurt am Main (DE); GENENTECH, INC., South San Francisco, CA 94080-4990 (US)
(72) Erfinder: Wehner, Volkmar, Dr., 97657 Sandberg (DE); Knolle, Jochen, Dr., 65830 Kriftel (DE); Stilz, Hans Ulrich, Dr., 65929 Frankfurt (DE); Gourvest, Jean-Francois, Dr., 77410 Claye Souilly (FR); Carniato, Denis, Dr., 91460 Marcoussis (FR); Gadek, Thomas, Richard, Dr., Oakland, CA 94611 (US); Mcdowell, Robert, Dr., San Francisco, CA 94114 (US); Pitti, Robert Maurice, El Cerrito, CA 94530 (US); Bodary, Sarah Carherine, Dr., San Bruno, CA 94066 (US)
(74) Vertreter: Vieillefosse, Jean-Claude

(56) Entgegenhaltungen:
- EP-A- 0 528 586
- EP-A- 0 528 587
- EP-A- 0 566 919
- WO-A-93/18057
- WO-A-94/08577
- WO-A-94/12181
- WO-A-95/14008
- WO-A-95/32710
- WO-A-96/00574
- WO-A-96/00730

## Beschreibung

Gegenstand der vorliegenden Erfindung sind Verbindungen der Formel I sowie deren physiologisch verträgliche Salze und solche Verbindungen enthaltende pharmazeutische Zubereitungen, deren Herstellung und Verwendung als Arzneimittel, insbesondere als Inhibitoren der Knochenresorption durch Osteoclasten, als Inhibitoren von Tumorwachstum und Tumormetastasierung, als Entzündungshemmer, zur Behandlung oder Prophylaxe von cardiovaskulären Erkrankungen wie Arteriosklerose oder Restenose, zur Behandlung oder Prophylaxe von Nephropathien und Retinopathien, wie z.B. diabetischer Retinopathie, sowie als Vitronectinrezeptor-Antagonisten zur Behandlung und Prophylaxe von Krankheiten, die auf der Wechselwirkung zwischen Vitronectinrezeptoren und deren Liganden bei Zell-Zell- oder Zell-Matrix-Interaktionsprozessen beruhen. Die Erfindung betrifft ferner die Verwendung der Verbindungen der Formel I sowie deren physiologisch verträglichen Salze und solche Verbindungen enthaltende pharmazeutische Zubereitungen als Arzeimittel zur Linderung oder Heilung von Krankheiten, die zumindest teilweise durch ein unerwünschtes Maß an Knochenresorption, Angiogenese, oder Proliferation von Zellen der glatten Gefäßmuskulatur bedingt sind.

Die menschlichen Knochen unterliegen einem fortwährenden dynamischen Umbauprozeß, der Knochenresorption und Knochenaufbau beinhaltet. Diese Prozesse werden von dafür spezialisierten Zelltypen gesteuert. Knochenaufbau beruht auf der Ablagerung von Knochenmatrix durch Osteoblasten, Knochenresorption beruht auf dem Abbau von Knochenmatrix durch Osteoclasten. Die Mehrzahl der Knochenerkrankungen beruhen auf einem gestörten Gleichgewicht zwischen Knochenbildung und Knochenresorption. Osteoporose ist charakterisiert durch einen Verlust an Knochenmatrix. Aktivierte Osteoclasten sind vielkernige Zellen mit einem Durchmesser bis zu 400 µm, die Knochenmatrix abtragen. Aktivierte Osteoclasten lagern sich an die Oberfläche der Knochenmatrix an und sezernieren proteolytische Enzyme und Säuren in die sogenannte "sealing zone", dem Bereich zwischen ihrer Zellmembran und der Knochenmatrix. Die saure Umgebung und die Proteasen bewirken den Abbau des Knochens.

Die erfindungsgemäßen Verbindungen der Formel inhibieren die Knochenresorption durch Osteoclasten. Knochenkrankheiten, gegen die die erfindungsgemäßen Verbindungen eingesetzt werden können, sind vor allem Osteoporose, Hypercalcämie, Osteopenie, z.B. hervorgerufen durch Metastasen, Zahnerkrankungen, Hyperparathyroidismus, periarticulare Erosionen bei rheumathoider Arthritis und Paget Krankheit.
Ferner können die Verbindungen der Formel I zur Linderung, Vermeidung oder Therapie von Knochenerkrankungen, die durch eine Glucocortikoid-, Steroid- oder Corticosteroid-Therapie oder durch einen Mangel an Sexualhormon(en) hervorgerufen werden, eingesetzt werden. Alle diese Erkrankungen sind durch Knochenverlust gekennzeichnet, der auf dem Ungleichgewicht zwischen Knochenaufbau und Knochenabbau beruht.

Studien haben gezeigt, daß die Anlagerung von Osteoclasten an den Knochen durch Integrin-Rezeptoren auf der Zelloberfläche von Osteoclasten gesteuert wird.

Integrine sind eine Superfamilie von Rezeptoren zu denen unter anderen der Fibrinogenrezeptor α_{IIb}β₃ auf den Blutplättchen und der Vitronectinrezeptor αᵥβ₃ gehören. Der Vitronectinrezeptor αᵥβ₃ ist ein membranständiges Glycoprotein, das auf der Zelloberfläche einer Reihe von Zellen wie Endothelzellen, Zellen der glatten Gefäßmuskulatur, Osteoclasten und Tumorzellen exprimiert wird. Der Vitronectinrezeptor αᵥβ₃, der auf der Osteoclastenmembran exprimiert wird, steuert den Prozeß der Anlagerung an den Knochen und der Knochenresorption und trägt somit zur Osteoporose bei.

α_{V}β₃ bindet hierbei an Knochenmatrixproteine wie Osteopontin, Knochensialoprotein und Thrombospontin, die das Tripeptidmotif Arg-Gly-Asp (oder RGD) enthalten.

Horton und Mitarbeiter beschreiben RGD-Peptide und einen anti-Vitronectinrezeptor Antikörper (23C6), die den Zahnabbau durch Osteoclasten und das Wandern von Osteoclasten inhibieren (Horton et al., Exp. Cell. Res. 1991, 195, 368). Sato et al. beschreiben in J. Cell Biol. 1990, 111, 1713 Echistatin, ein RGD-Peptid aus Schlangengift, als potenten Inhibitor der Knochenresorption in einer Gewebekultur und als Hemmstoff der Osteoclasten-Anheftung an den Knochen. Fischer et al. (Endocrinology, 1993, 132, 1411) konnten an der Ratte zeigen, daß Echistatin die Knochenresorption auch in vivo hemmt.

Der Vitronectinrezeptor αᵥβ₃ auf humanen Zellen der glatten Gefäßmuskulatur der Aorta stimuliert die Wanderung dieser Zellen in das Neointima, was schließlich zu Arteriosklerose und Restenose nach Angioplastie führt (Brown et al., Cardiovascular Res. 1994, 28, 1815).

Die Verbindungen der Formel I können ferner als Träger von Wirkstoffen dienen, um den Wirkstoff gezielt an den Wirkort zu transportieren (= Drug Targeting, siehe z.B. Targeted Drug Delivery, R.C. Juliano, Handbook of Experimental Pharmacology Vol. 100, Ed. Born, G.V.R. et al., Springer Verlag). Bei den Wirkstoffen handelt es sich um solche, die zur Behandlung der obengenannten Krankheiten verwendet werden können.

Brooks et al. (Cell 1994, 79, 1157) zeigten, daß Antikörper gegen αᵥβ₃ oder α_{V}β₃-Antagonisten eine Schrumpfung von Tumoren bewirken können, indem sie die Apoptose von Blutgefäßzellen während der Angiogenese induzieren. Chersh et al. (Science 1995, 270, 1500) beschreiben anti αᵥβ₃-Antikörper oder αᵥβ₃-Antagonisten, die bFGF induzierte Angiogeneseprozesse im Rattenauge inhibieren, was therapeutisch bei der Behandlung von Retinopathien nützlich sein könnte.

In der Patentanmeldung WO 94/12181 werden substituierte aromatische oder nichtaromatische Ringsysteme, in WO 94/08577 substituierte Heterocyclen als Fibrinogenrezeptor-Antagonisten und Inhibitoren der Plättchenaggregation beschrieben. Aus EP-A-518 586 und EP-A-528 587 sind Aminoalkyl- oder Heterocyclyl-substituierte Phenylalanin-Derivate, aus WO 95/32710 Arylderivate als Hemmstoffe der Knochenresorption durch Osteoclasten bekannt. Die WO 96/00574 beschreibt Benzodiazepine, die WO 96/00730 Fibrinogenrezeptorantagonisten-Template, insbesondere Benzodiazepine, die an einen Stickstoff tragenden 5-Ring geknüpft sind, als Vitronectinrezeptor-Antagonisten.

Gegenstand der vorliegenden Erfindung sind Cycloalkyl-Derivate der Formel I,

R¹-Y-A-B-D-E-F-G I,

worin bedeuten:
- A: -NH-C(O)- ;
- B: (C₁-C₄)-Alkandiyl;
- D: -O-, -NR²-C(O)-, -C(O)-NR²- oder eine direkte Bindung;
- E: Phenylen oder Pyridindiyl;
- F: -CH₂- oder -C(O)NHCH₂- ;
- G: 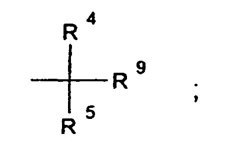
- Y: eine direkte Bindung;
- R¹: H₂N-C(=NH)-, 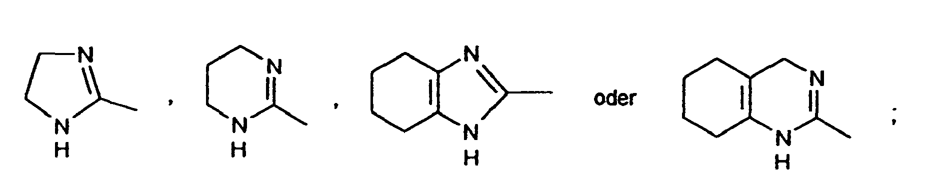
- R²: H oder (C₁-C₄)-Alkyl;
- R⁴: R⁶OC(O)NH-;
- R⁵: H;
- R⁶: Adamantyl-1-(C₁-C₃)-alkylen, Adamantyl-2-(C₁-C₃)-alkylen, 1-Adamantyl, 2-Adamantyl, wobei Adamantyl bevorzugt 1 oder 2fach durch (C₁-C₄)-Alkyl, Trifluormethyl, Phenyl, Benzyl, (C₁-C₄)-Alkoxy, Phenoxy oder Benzyloxy substituiert ist, oder (C₁₁-C₁₂)-Cycloalkyl, das wie vorstehend 1- oder 2-fach substituiert sein kann;
- R⁹: C(O)R¹⁰;
- R¹⁰: OH, (C₁-C₆)-Alkoxy, Phenoxy, Benzyloxy oder (C₁-C₄)-Alkoxycarbonyloxy-(C₁ -C₄)-alkandiyloxy;
in allen ihren stereoisomeren Formen und Gemische davon in allen Verhältnissen, und ihre physiologisch verträglichen Salze.

Die in den Substituenten auftretenden Alkylreste können geradkettig oder verzweigt, gesättigt oder einfach oder mehrfach ungesättigt sein. Entsprechendes gilt für davon abgeleitete Reste, wie z.B. Alkoxy.

Physiologisch verträgliche Salze der Verbindungen der Formel I sind insbesondere pharmazeutisch verwendbare oder nicht-toxische Salze. Solche Salze werden beispielsweise von Verbindungen der Formel I, welche saure Gruppen, z. B. Carboxy, enthalten, mit Alkali- oder Erdalkalimetallen gebildet, wie z.B. Na, K, Mg und Ca, sowie mit physiologisch verträglichen organischen Aminen, wie z.B. Triethylamin, Ethanolamin oder Tris-(2-hydroxy-ethyl)-amin. Verbindungen der Formel I, weiche basische Gruppen, z.B. eine Aminogruppe, eine Amidinogruppe oder eine Guanidinogruppe enthalten, bilden mit anorganischen Säuren, wie z.B. Salzsäure, Schwefelsäure oder Phosphorsäure, und mit organischen Carbon- oder Sulfonsäuren, wie z.B. Essigsäure, Citronensäure, Benzoesäure, Maleinsäure, Fumarsäure, Weinsäure, Methansulfonsäure oder p-Toluolsulfonsäure, Salze.

Die erfindungsgemäßen Verbindungen der Formel I können optisch aktive Kohlenstoffatome, die unabhängig voneinander R- oder S-Konfiguration haben können, enthalten und somit in Form reiner Enantiomerer oder reiner Diastereomerer oder in Form von Enantiomerengemischen oder Diastereomerengemischen vorliegen. Sowohl reine Enantiomere und Enantiomerengemische als auch Diastereomere und Diastereomerengemische sind Gegenstand der vorliegenden Erfindung. Die Erfindung umfaßt Gemische von zwei Stereoisomeren und von mehr als zwei Stereoisomeren der Formel I und alle Verhältnisse der Stereoisomeren in den Gemischen.

Die erfindungsgemäßen Verbindungen der Formel I können, falls A, D oder F unabhängig voneinander -CR²=CR³- sind, als E/Z-Isomerengemische vorliegen. Gegenstand der vorliegenden Erfindung sind sowohl reine E- bzw. Z-Isomere als auch Gemische von E/Z-Isomeren in allen Verhältnissen. Diastereomere einschließlich E/Z-Isomere können durch Chromatographie in die Einzelisomeren aufgetrennt werden. Racemate können entweder durch Chromatographie an chiralen Phasen oder durch Racematspaltung in die beiden Enantiomere aufgetrennt werden.
Die erfindungsgemäßen Verbindungen der Formel I können darüber hinaus bewegliche Wasserstoffatome enthalten, also in verschiedenen tautomeren Formen vorliegen. Auch diese Tautomeren sind Gegenstand der vorliegenden Erfindung.

Bevorzugt sind Verbindungen der Formel I,
(2S)-2-(1-Adamantylmethyloxycarbonylamino)-3-(4-(3-guanidinocarbonyl-propyloxy)phenyl)-propionsäure
(2S)-2-((2-(1-Adamantyl)-ethyl)oxycarbonylamino)-3-(4-(3-guanidinocarbonyl-propyloxy)phenyl)-propionsäure
(2S)-2-(1-Adamantylmethyloxycarbonylamino)-3-(4-(2-(1,4,5,6-tetrahydropyrimidin-2-yl-carbamoyl)-ethyl) benzoylamino)-propionsäure
(2S)-2-(1-Adamantylmethyloxycarbonylamino)-3-(4-(3-(1,4,5,6-tetrahydropyrimidin-2-yl-carbamoyl)-propyloxy)phenyl)-propionsäure
(2S)-2-(1-Adamantylmethyloxycarbonylamino)-3-(4-(3-(4,5-dihydroimidazol-2-yl-carbamoyl)-propyloxy)phenyl)-propionsäure

Verbindungen der Formel I können generell, beispielsweise im Zuge einer konvergenten Synthese, durch Verknüpfung zweier oder mehrerer Fragmente, die sich retrosynthetisch aus der Formel I ableiten lassen, hergestellt werden. Bei der Herstellung der Verbindungen der Formel I kann es generell im Laufe der Synthese nötig sein, funktionelle Gruppen, die im jeweiligen Syntheseschritt zu unerwünschten Reaktionen oder Nebenreaktionen führen könnten, durch eine dem Syntheseproblem angepaßte Schutzgruppenstrategie temporär zu blockieren, was dem Fachmann bekannt ist. Die Methode der Fragmentverknüpfung ist nicht auf die nachfolgenden Beispiele beschränkt, sondern allgemein für Synthesen der Verbindungen der Formel 1 anwendbar.

Beispielsweise können Verbindungen der Formel 1 des Typs,

R¹-Y-A-B-D-E-C(O)NR²-G,

in dem F in der Formel I für -C(O)NR²- steht' durch Kondensation einer Verbindung der Formel II,

R¹-Y-A-B-D-E-M II,

wobei M für Hydroxycarbonyl, (C₁-C₆)-Alkoxycarbonyl, aktivierte Carbonsäurederivate wie Säurechloride, Aktivester oder gemischte Anhydride steht, mit HNR²-G hergestellt werden.

Zur Kondensation zweier Fragmente unter Bildung einer Amidbindung verwendet man vorteilhafterweise die an sich bekannten Kupplungsmethoden der Peptidchemie (siehe z.B. Houben-Weyl, Methoden der Organischen Chemie, Band 15/1 und 15/2, Georg Thieme Verlag, Stuttgart, 1974). Dazu ist es in der Regel nötig, daß vorhandene, nicht reagierende Aminogruppen durch reversible Schutzgruppen während der Kondensation geschützt werden. Gleiches gilt für nicht an der Reaktion beteiligte Carboxylgruppen, die bevorzugt als (C₁-C₆)-Alkyl-, Benzyl- oder tert.-Butylester eingesetzt werden. Ein Aminogruppen-Schutz erübrigt sich, wenn die zu generierenden Aminogruppen noch als Nitro- oder Cyanogruppen vorliegen und erst nach der Kupplung durch Hydrierung gebildet werden. Nach der Kupplung werden die vorhandenen Schutzgruppen in geeigneter Weise abgespalten. Beispielsweise können NO₂-Gruppen (Guanidinoschutz), Benzyloxycarbonylgruppen und Benzylester abhydriert werden. Die Schutzgruppen vom tert-Butyltyp werden sauer abgespalten, während der 9-Fluorenylmethyloxycarbonylrest durch sekundäre Amine entfernt wird.

Die Verbindungen der Formel I und ihre physiologisch verträglichen Salze können am Tier, bevorzugt am Säugetier, und insbesondere am Menschen als Arzneimittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Salzes davon, neben üblichen pharmazeutisch einwandfreien Träger- und Zusatzstoffen enthalten. Die Zubereitungen enthalten normalerweise etwa 0,5 bis 90 Gew.-% der therapeutisch wirksamen Verbindung.

Die Arzneimittel können oral, z.B. in Form von Pillen, Tabletten, Lacktabletten, Dragees, Granulaten, Hart- und Weichgelatinekapseln, Lösungen, Sirupen, Emulsionen, Suspensionen oder Aerosolmischungen verabreicht werden. Die Verabreichung kann aber auch rektal, z.B. in Form von Suppositorien, oder parenteral, z.B. in Form von Injektions- oder Infusionslösungen, Mikrokapseln oder Rods, perkutan, z.B. in Form von Salben oder Tinkturen, oder nasal, z.B. in Form von Nasalsprays, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B.Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln, Implantate oder Rods eignen sich Mischpolymerisate aus Glykolsäure und Milchsäure.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder deren physiologisch verträglichen Salze enthalten; ferner neben mindestens einer Verbindung der Formel I noch einen oder mehrere andere therapeutisch wirksame Stoffe.

Die Dosis kann innerhalb weiter Grenzen variieren und ist in jedem einzelnen Fall den individuellen Gegebenheiten anzupassen. Bei oraler Verabreichung beträgt die Tagesdosis im allgemeinen 0,01 bis 50 mg/kg, vorzugsweise 0,1 bis 5 mg/kg, insbesondere 0,3 bis 0,5 mg/kg Körpergewicht zur Erzielung wirksamer Ergebnisse. Bei intravenöser Applikation beträgt die Tagesdosis im allgemeinen etwa 0,01 bis 100 mg/kg, vorzugsweise 0,05 bis 10 mg/kg Körpergewicht. Die Tagesdosis kann, insbesondere bei der Applikation größerer Mengen, in mehrere, z.B. 2, 3 oder 4, Teilverabreichungen aufgeteilt werden. Gegebenenfalls kann es, je nach individuellem Verhalten, erforderlich werden, von der angegebenen Tagesdosis nach oben oder nach unten abzuweichen.

### Beispiele

Die Produkte wurden über Massenspektren und/oder NMR-Spektren identifiziert.

### Beispiel 1

### (2S)-2-(1-Adamantylmethyloxycarbonylamino)-3-(4-(3-guanidinocarbonyl-propyloxy)phenyl)-propionsäure (1.5)

Die Synthese erfolgte nach folgender Reaktionssequenz:

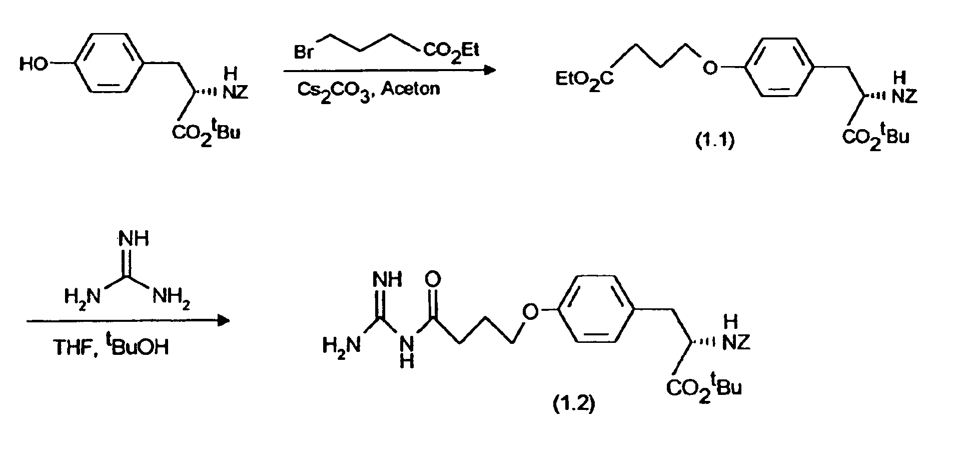

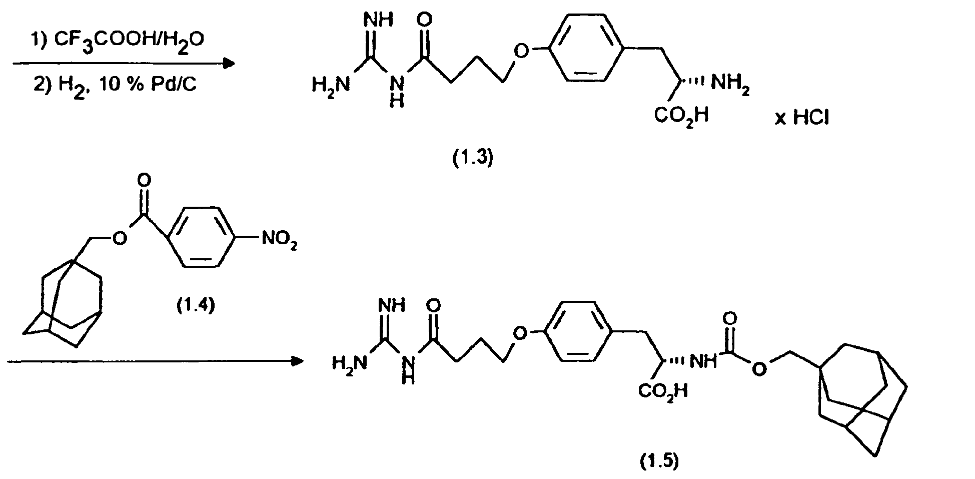

### 1a) (2S)-2-Benzyloxycarbonylamino-3-(4-(3-ethoxycarbonyl-propyloxy)phenyl)-propionsäure-tert.-butylester (1.1)

Man gab zu 21,5 g (57,9 mMol) N-Benzyloxycarbonyl-tyrosin-tert.-butylester in 280 ml Aceton 8,29 ml (57,9 mMol) 4-Brombutansäure-ethylester und 28,21 g (86,58 mMol) Cäsiumcarbonat und erhitzte unter Rühren zum Rückfluß. Nach 2h wurden nochmals 2 ml 4-Brombutansäure-ethylester und 2 g Cäsiumcarbonat, nach weiteren 2 h weitere 2 ml 4-Brombutansäure-ethylester und 3 g Cäsiumcarbonat und nach Stehen bei Raumtemperatur über Nacht nochmals 9 ml 4-Brombutansäureethylester zugegeben und das Gemisch weitere 6 h zum Rückfluß erhitzt. Nach Abkühlen wurde filtriert, der Rückstand mit Aceton gewaschen und das Filtrat eingeengt. Der Rückstand wurde in Diethylether aufgenommen und die organische Phase nacheinander mit 3 %iger Zitronensäurelösung, 3 x H₂O und gesättigter NaCI-Lösung gewaschen. Die Etherphase wurde über MgSO₄ getrocknet, das Trockenmittel abfiltriert und das Filtrat im Vakuum eingeengt. Der Rückstand wurde mit CH₂Cl₂ und CH₂Cl₂MeOH (99/1) über Kieselgel chromatographiert. Man erhielt 31,3 g eines blaßgelben Öls, das ohne weitere Reinigung zur Synthese von (1.2) eingesetzt wurde.

### 1b) (2S)-2-Benzyloxycarbonylamino-3-(4-(3-guanidinocarbonyl-propyloxy)phenyl)-propionsäure-tert.-butylester (1.2)

Man gab zu einer Lösung von 20 g (41,23 mMol) (1.1) in THF eine Lösung von 3,64 g (61,69 mMol) Guanidin in 150 ml tert. Butanol und rührte 18 h bei Raumtemperatur. Anschließend gab man weitere 4,5 g Guanidin in 150 ml tert. Butanol zu, rührte 7 h bei Raumtemperatur, engte die Reaktionslösung auf ca. die Hälfte ein und rührte weitere 18 h bei Raumtemperatur. Das Lösungmittel wurde im Vakuum entfernt und der Rückstand zunächst mit CH₂Cl₂/MeOH/H₂O (95/5/0,5) über basisches Al₂O₃ filtriert und anschließend mittels MPLC mit CH₂Cl₂/MeOH/Essigsäure (90/10/0,5) über Kieselgel chromatographiert. Man erhielt 8,6 g (42 %) an (1.2).

### 1c) (2S)-2-Amino-3-(4-(3-guanidinocarbonyl-propyloxy)phenyl)-propionsäure-Hydrochlorid (1.3)

Man gab zu 8,6 g (17,3 mmol) (1.2) 30 ml 95 %ige Trifluoressigsäure und ließ 25 min bei Raumtemperatur rühren. Das Reaktionsgemisch wurde einrotiert und anschließend zweimal mit Toluol eingeengt. Der Rückstand wurde in verdünnter Essigsäure aufgenommen, mit Wasser versetzt und gefriergetrocknet. Der so erhaltene farblose Feststoff wurde mittels MPLC mit CH₂Cl₂/MeOH/Essigsäure (90/10/0,5) über Kieselgel gereinigt. Nach Einengen und Gefriertrocknen erhielt man 5,5 g (72 %) eines farblosen Feststoffs.

400 mg dieser Substanz wurden in 30 ml MeOH gelöst und nach Zusatz von methanolischer Salzsäure die Benzyloxycarbonylschutzgruppe über 10 % Pd/C hydrogenolytisch gespalten. Das ausgefallene Produkt wurde durch Zugabe von DMF in Lösung gebracht, der Katalysator wurde abfiltriert, das Filtrat eingeengt und der Rückstand gefriergetrocknet. Man erhielt 320 mg an (1.3) als farblosen Feststoff.

### 1d) (1-Adamantylmethyl)-4-nitrophenyl-carbonat (1.4)

Man gab zu einer Lösung von 499 mg (3 mMol) 1-Hydroxymethyl-adamantan in 7 ml Pyridin 605 mg (3 mmol) Chlorameisensäure-4-nitrophenylester und ließ über Nacht bei Raumtemperatur rühren. Nach Einengen im Hochvakuum wurde der Rückstand direkt zur Herstellung von (1.5) eingesetzt.

### 1e) (2S)-2-(1 -Adamantylmethyloxycarbonylamino)-3-(4-(3-guanidinocarbonyl-propyloxy)phenyl)-propionsäure (1.5)

Man gab zu einer Lösung von 146 mg (0,35 mMol) (1.3) in 2 ml DMF 114,5 mg (1.4) und rührte über Nacht bei Raumtemperatur. Man gab 0,059 ml Diisopropylethylamin zu und rührte erneut über Nacht bei Raumtemperatur. Nach Entfernen des Lösungsmittels im Vakuum wurde der Rückstand zwischen Essigester und Wasser verteilt. Die organische Phase wurde über MgSO₄ getrocknet, konzentriert und anschließend mit Diisopropylether versetzt. Der Niederschlag wurde abfiltriert und mittels präparativer Dünnschichtchromatographie mit CH₂Cl₂/MeOH/Essigsäure (100/25/2) gereinigt. Man erhielt 10 mg an (1.5).

### Beispiel 2

### (2S)-2-((2-(1-Adamantyl)-ethyl)oxycarbonylamino)-3-(4-(3-guanidinocarbonyl-propyloxy)phenyl)-propionsäure (2.2)

Die Synthese erfolgte nach folgender Reaktionssequenz:

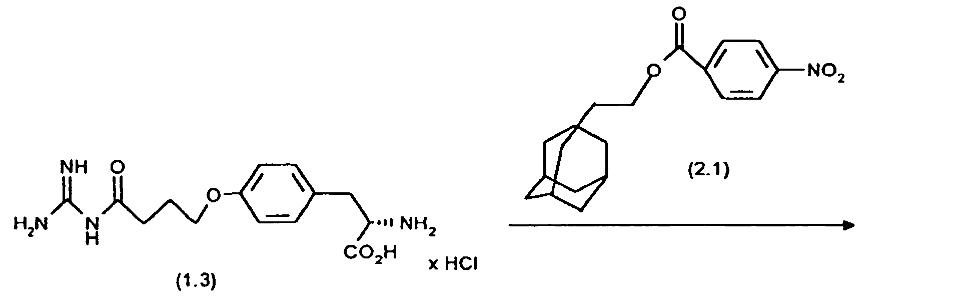

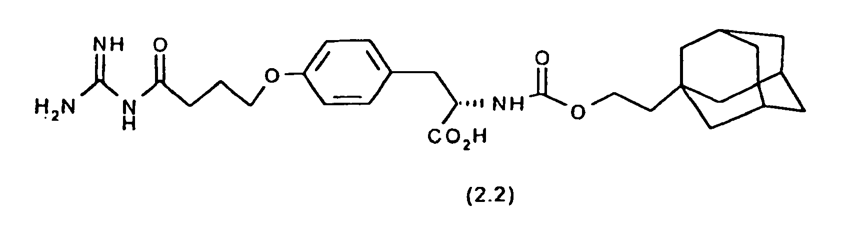

Die Synthese von (1.3) erfolgte wie in Beispiel 1, 1c) beschrieben. (2.1) wurde analog (1.4) (Beispiel 1, 1d)) aus 1-(2-Hydroxyethyl)adamantan und Chlorameisensäure-4-nitrophenylester hergestellt und direkt zur Synthese von (2.2) eingesetzt.

### (2S)-2-((2-(1-Adamantyl)-ethyl)oxycarbonylamino)-3-(4-(3-guanidinocarbonyl-propyloxy)phenyl)-propionsäure (2.2)

Man gab zu einer Lösung von 146 mg (0,35 mmol) (1.3) in 2 ml DMF 119 mg (2.1) und rührte über Nacht bei Raumtemperatur. Man gab 2,3 mg Imidazol und 0,3 ml Pyridin zu und rührte erneut über Nacht bei Raumtemperatur. Die Lösung wurde eingeengt, der Rückstand zwischen Wasser und Essigester verteilt, die organische Phase über MgSO₄ getrocknet und nach Filtration das Lösungsmittel im Vakuum entfernt. Der Rückstand wurde mittels präparativer Dünnschichtchromatographie mit CH₂Cl₂/MeOH/Essigsäure (100/25/2) aufgetrennt. Man erhielt 19 mg an (2.2).

### Beispiel 3

### (2S)-2-(1-Adamantylmethyloxycarbonylamino)-3-(4-(2-(1,4,5,6-tetrahydropyrimidin-2-yl-carbamoyl)-ethyl)benzoylamino)-propionsäure (g)

Die Synthese erfolgte nach folgender Reaktionssequenz

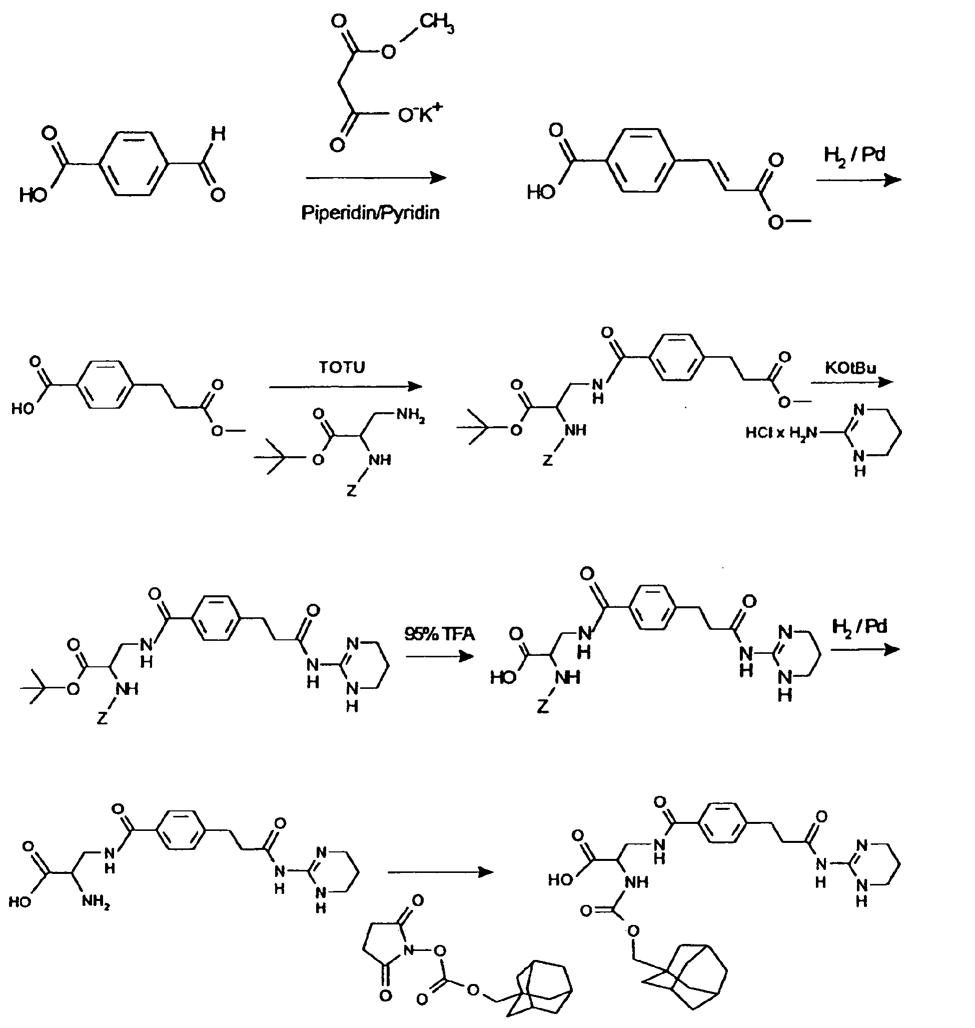

### a) 4-(2-Methoxycarbonylvinyl)benzoesäure

18,74 g (0,12 mol) Malonsäuremonomethylester-Kaliumsalz wurden in 18 ml Pyridin suspendiert. Unter Rühren wurden bei Raumtemperatur (RT) 15,01 g (0,1 mol) 4-Carboxybenzaldehyd und 0,85 g (0,01 mol) Piperidin zugegeben und bis zur Beendigung der CO₂ -Entwicklung (ca. 2h) unter Rückfluß gekocht. Es wurden weitere 60 ml Pyridin zugegeben und noch 1 h bei Rückfluß nachgerührt. Das Reaktionsgemisch wurde unter Rühren mit 500 ml Eis und 110 ml konz. HCl versetzt. Nach beendeter Zugabe wurde noch 20 min gerührt, das Produkt abgesaugt, mit Wasser gewaschen und aus Isopropanol umkristallisiert. Ausbeute: 12,85 g (62%).
¹H-NMR (200 MHz, DMSO) : δ = 3,75 (s, 3H, OCH₃); 6,76 (d, J = 15 Hz, 1H, CHCOOCH₃); 7,73 (d, J = 15 Hz, 1H, Ar-CH); 7,84 (d, J = 9 Hz, 2H, Ar-H); 7,98 (d, J = 9 Hz, 2H, Ar-H); 13,11 (s, breit, 1 H, COOH)
MS: CI⁺, m/e = 207,2 (M+H⁺, 100%)
HPLC: (RP18: Nucleosil 300-5-C18, 250 x 4 mm), Puffer A: H₂O, 0,1% TFA; Puffer B: Acetonitril (80% v/v); H₂O (20% v/v): 0,1% TFA; Gradient: (1) 5 min, 10% Puffer B; (2) während 20 min auf 90% Puffer B; (3) 5 min 90% Puffer B; Fluß 1ml/min; Rt = 18,05 min.

### b) 4-(2-Methoxycarbonylethyl)benzoesäure

8 g (38,8 mmol) 4-(2-Methoxycarbonylvinyl)benzoesäure (Beispiel a) wurden in 250 ml Dioxan suspendiert und 7h bei RT über Pd/C (10%) mit 1 bar H₂ hydriert. Es wurde filtriert und das Lösungsmittel im Vakuum abgedampft. Ausbeute: 8,05 g (100%).
¹H-NMR (200 MHz, DMSO): δ = 2,67 (t, J = 8 Hz, 2H, CH₂-COOMe); 2,93 (t, J = 8 Hz, 2H, Ar-CH₂); 3,59 (s, 3H, OCH₃); 7,35 (d, 2H, Ar-H); 7,86 (d, J = 9 Hz, 2H, Ar-H); 12,80 (s, breit, 1H, COOH)
MS: CI⁺, m/e = 209,2 (M+H⁺, 100%)
HPLC: (RP18: Nucleosil 300-5-C18, 250 x 4 mm), Puffer A: H₂O, 0,1% TFA; Puffer B: Acetonitril (80% v/v); H₂O (20% v/v); 0,1% TFA; Gradient: (1) 5 min, 10% Puffer B; (2) während 20 min auf 90% Puffer B; (3) 5 min 90% Puffer B; Fluß 1ml/min; Rt = 17,03 min.

### c) (2S)-2-Benzyloxycarbonylamino-3-(4-(2-methoxycarbonylethyl)benzoylamino)-propionsäure-tert.-butylester

354 mg (1,7 mmol) 4-(2-Methoxycarbonylethyl)benzoesäure (Beispiel b) und 500 mg (1,7 mmol) (2S)-2-Benzyloxycarbonylamino-3-amino-propionsäure-tert.-butylester wurden in 3 ml DMF gelöst und mit 557 mg (1,7 mmol) O-[(Cyano-(ethoxycarbonyl)methyliden)amino]-1,1,3.3,-tetramethyluroniumtetrafluorborat (TOTU) und 204 mg (1,7 mmol) Diisopropylethylamin (DIPEA) versetzt und 7h bei RT gerührt. Das Lösungsmittel wurde im Vakuum abgedampft, der Rückstand in Essigsäureethylester (EE) gelöst und je dreimal mit KHSO₄ und NaHCO₃-Lösung neutral gewaschen, die organische Phase wurde getrennt, getrocknet und das Lösungsmittel im Vakuum abdestilliert, Ausbeute : 770 mg (93%).
MS: ES⁺, m/e = 485,2 (M+H⁺, 100%)

### d) (2S)-2-Benzyloxycarbonylamino-3-(4-(2-(1,4,5,6-tetrahydropyrimidin-2-yl-carbamoyl)-ethyl)benzoylamino)-propionsäure-tert.-butylester

1,25 g (9,2 mmol) 2-Amino-1,4,5,6-tetrahydropyrimidin-Hydrochlorid und 1,03 g (9,2 mmol) Kaliumtert.-butylat wurden in 3 ml absol. DMF gelöst und 30 min bei RT gerührt. Dann wurden 740 mg (1,53 mmol) (2S)-2-Benzyloxycarbonylamino-3-(4-(2-methoxycarbonylethyl)-benzoylamino)-propionsäure-tert-butylester (Beispiel c) in 1 ml DMF zugegeben und es wurde 4h bei RT gerührt. Es wurde mit Eisessig auf pH 4 eingestellt, das Lösungsmittel im Vakuum abgedampft, der Rückstand über Kieselgel (Dichlormethan/Methanol/Eisessig/Wasser (9/1/0,1/0,1)) chromatographiert. Ausbeute: 190 mg (38%).
MS: ES⁺, m/e = 552,3 (M+H⁺, 100%)

### e) (2S)-2-Benzyloxycarbonylamino-3-(4-(2-(1,4,5,6-tetrahydropyrimidin-2-yl-carbamoyl)-ethyl)benzoylamino)-propionsäure

190 mg (0,34 mmol) (2S)-2-Benzyloxycarbonylamino-3-(4-(2-(1,4,5,6-tetrahydropyrimidin-2-yl-carbamoyl)-ethyl)benzoylamino)-propionsäure-tert.-butylester (Beispiel d) wurden in 5 ml 95% Trifluoressigsäure gelöst und 1 h bei RT gerührt. Die Trifluoressigsäure wurde im Vakuum abdestilliert, es wurde mit Toluol koevaporiert, der Rückstand in Eisessig gelöst, mit Wasser verdünnt und gefriergetrocknet. Ausbeute: 170 mg (100%).
MS: ES⁺, m/e = 496,3 (M+H⁺, 100%)

### f) (2S)-2-Amino-3-(4-(2-(1,4,5,6-tetrahydropyrimidin-2-yl-carbamoyl)-ethyl)benzoylamino)-propionsäure

100 mg (0,2 mmol) (2S)-2-Benzyloxycarbonylamino-3-(4-(2-(1,4,5,6-tetrahydropyrimidin-2-yl-carbamoyl)-ethyl)benzoylamino)-propionsäure (Beispiel e) wurden in 15 ml Dioxan gelöst, mit 0,012 ml Eisessig versetzt und über Pd/C (5%) bei RT und 1 bar H₂ hydriert. Nach 2h wurden 15 ml Methanol zugegeben und weitere 5h bei RT und 1 bar H₂ hydriert. Es wurde filtriert und das Lösungsmittel im Vakuum abgedampft. Ausbeute: 67,4 mg (93%).
MS: ES⁺, m/e = 362,2 (M+H⁺, 30%); 173,0 (100).

### g) (2S)-2-(1-Adamantylmethyloxycarbonylamino)-3-(4-(2-(1,4,5,6-tetrahydropyrimidin-2-yl-carbamoyl)-ethyl)benzoylamino)-propionsäure

67,4 mg (0,186 mmol) (2S)-2-Amino-3-(4-(2-(1,4.5,6-tetrahydropyrimidin-2-yl-carbamoyl)-ethyl)benzoyl-amino)-propionsäure (Beispiel f) wurden in 4 ml Dioxan gelöst. Unter Rühren wurden bei RT zuerst 4 ml gesättigte NaHCO₃-Lösung, dann 57 mg Kohlensäure-(1-adamantylmethyl)ester-(2,5-dioxo-pyrrolidin-1-yl)ester zugegeben. Es wurde 24h bei RT gerührt, mit Eisessig auf pH 4 gestellt, das Lösungsmittel im Vakuum abgedampft und der Rückstand über RP-18 (Lichrospher C-18) chromatographiert (20 % (v/v) Acetonitril in Wasser, 0, 1 % Trifluoressigsäure, bis zu 40%(v/v) Acetonitril). Ausbeute: 30 mg (30%).
MS: ES⁺, m/e = 554,4 (M+H⁺, 100%).

### Beispiel 4

### (2S)-2-(1-Adamantylmethyloxycarbonylamino)-3-(4-(3-(1,4,5,6-tetrahydropyrimidin-2-yl-carbamoyl)-propyloxy)phenyl)-propionsäure

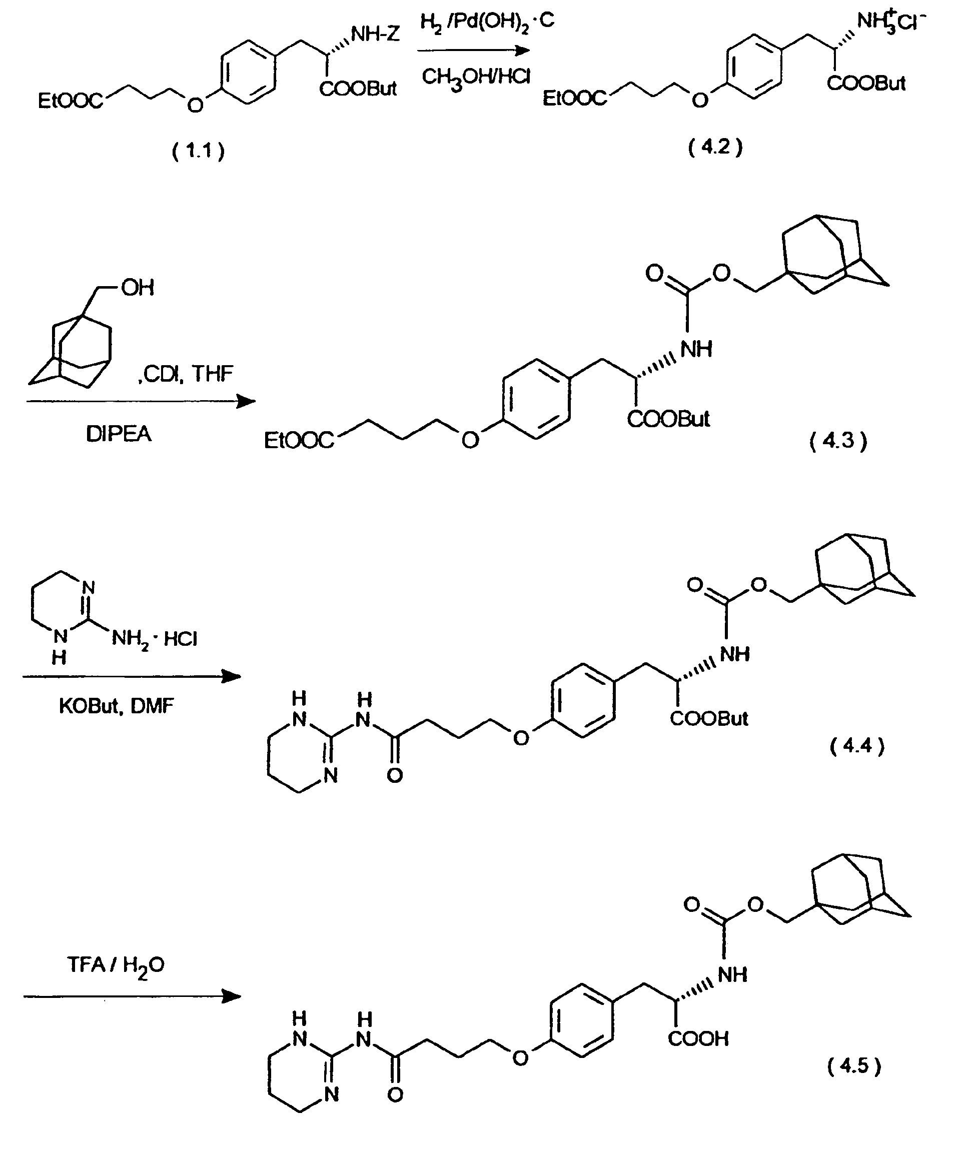

### a) (2S)-2-Amino-3-(4-(3-ethoxycarbonyl-propyloxy)phenyl)-propionsäure-tert.-butylester-Hydrochlorid (4.2)

Man löste 100 g (0,206 mol) (2S)-2-Benzyloxycarbonylamino-3-(4-(3-ethoxycarbonyl-propyloxy)phenyl)-propionsäure-tert.-butylester (1.1) aus Beispiel 1 in 1 Liter Methanol, versetzte mit methanolischer Salzsäure und mit 10 g 20 % Palladiumhydroxid/Kohle und leitete für 6 Stunden Wasserstoff ein. Dann wurde vom Katalysator abfiltriert, die Lösung eingeengt und der Rückstand mit tert.-Butylether versetzt. Der entstandene Niederschlag wurde abgesaugt. Man erhielt man 72 g (90%) eines amorphen Pulvers.

### b) (2S)-2-(1 -Adamantylmethyloxycarbonylamino)-3-(4-(3-ethoxycarbonyl-propyloxy)phenyl)-propionsäure-tert.-butylester (4.3)

Man gab zu einer Lösung von 830 mg (5 mmol) 1-Hydroxymethyl-adamantan in 10 ml Tetrahydrofuran 892 mg (5,5 mmol) Carbonyldiimidazol (CDI) und ließ über Nacht bei RT rühren. Anschließend versetzte man mit 1 g (2,57 mmol) (2S)-2-Amino-3-(4-(3-ethoxycarbonyl-propyloxy)phenyl)-propionsäure-tert.-butylester-Hydrochlorid und 442 µl (2,57 mmol) Diisopropylethylamin (DIPEA) und ließ über Nacht bei 50°C rühren. Nach Abkühlen wurde in Ethylacetat aufgenommen und die organische Phase nacheinander mit 3%iger Zitronensäurelösung, Natriumhydrogencarbonatlösung, 3xH₂O und gesättigter NaCl-Lösung gewaschen. Die organische Phase wurde mit MgSO₄ getrocknet, das Trockenmittel abfiltriert und das Filtrat eingeengt. Der Rückstand wurde mit CH₂Cl₂ / CH₃OH (99/1) über Kieselgel chromatographiert. Man erhielt 1,19 g (85 %) eines Öls, das ohne weitere Reinigung zur Synthese von (4.4) eingesetzt wird.

### c) (2S)-2-(1-Adamantylmethyloxycarbonylamino)-3-(4-(3-(1,4,5,6-tetrahydropyrimidin-2-yl-carbamoyl)-propoxy)phenyl)-propionsäure-tert.-butylester (4.4)

398 mg (2,94 mmol) 2-Amino-1,4,5,6-tetrahydropyrimidin-Hydrochlorid wurden in 7 ml Methanol gelöst und mit 330 mg (2,94 mmol) Kalium-tert.-butylat versetzt. Nach 40 min filtrierte man von den ausgefallenen Salzen ab und engte das Filtrat ein. Der Rückstand wurde in 3 ml Dimethylformamid gelöst und zu einer Lösung von 365 mg (2S)-2-(1-Adamantylmethyloxycarbonylamino)-3-(4-(3-ethoxycarbonyl-propyloxy)phenyl)-propionsäure-tert.-butylester (4.3) gegeben. Man erwärmte für 5 Stunden auf 40°C, entfernte das Lösungsmittel im Vakuum, nahm in Ethylacetat auf und wusch die organische Phase 3x mit H₂O und gesättigter Natriumchlorid-Lösung. Die organische Phase wurde eingeengt und der Rückstand mit CH₂Cl₂/CH₃OH/Ethylacetat/H₂O (90:10:0,5:0,5) über Kieselgel chromatographiert. Man erhielt 100 mg eines amorphen Pulvers, das ohne weitere Reinigung zur Synthese von (4.5) eingesetzt wurde.

### d) (2S)-2-(1-Adamantylmethyloxycarbonylamino)-3-(4-(3-(1,4,5,6-tetrahydropyrimidin-2-yl-carbamoyl)-propyloxy)-propionsäure (4.5)

100 mg des tert-Butylesters aus Beispiel 4.4 wurden in 10 ml Trifluoressigsäure/H₂O (95:5) gelöst. Nach 30 min wurde die Reaktionslösung eingeengt und der Rückstand wurde mit Diisopropylether digeriert. Durch anschließende Gefriertrocknung erhielt man 85 mg (4.5).

### Beispiel 5

### (2S)-2-(1-Adamantylmethyloxycarbonylamino)-3-(4-(3-(4,5-dihydroimidazol-2-yl-carbamoyl)-propyloxy)phenyl)-propionsäure (5.2)

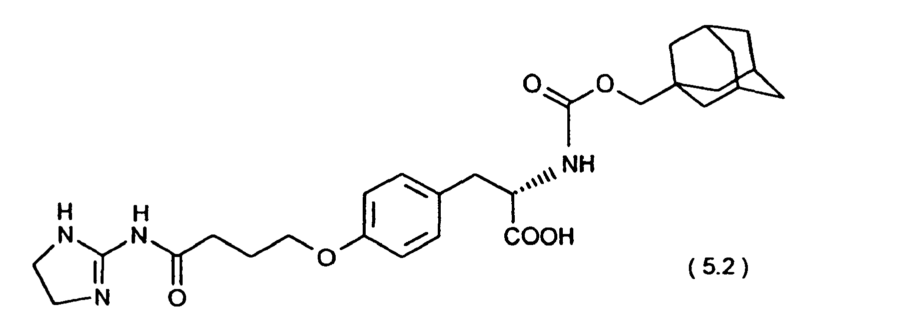

Die Herstellung erfolgte analog Beispiel 4.

### a) (2S)-2-(1 -Adamantylrnethyloxycarbonylamino)-3-(4-(3-(4,5-dihydromidazol-2-yl-carbamoyl)-propyloxy)phenyl)-propionsäure-tert.-butylester (5.1)

436 mg (0,8 mmol) (2S)-2-(1-Adamantylmethyloxycarbonylamino)-3-(4-(3-ethoxycarbonyl-propyloxy)phenyl)-propionsäure-tert.-butylester wurden zu einer Lösung von 388 mg (3,2 mmol) 2-Amino-4,5-dihydroimidazol-Hydrochlorid und 359 mg (3,2 mmol) Kalium-tert.-butylat in 10 ml DMF gegeben. Man ließ über Nacht rühren. Nach Beendigung der Reaktion wurde analog dem Beispiel 4c aufgearbeitet und mit dem gleichen Laufmittelgemisch über Kieselgel chromatographiert. Man erhielt 188 mg (0,32 mmol) (5.1).

### b) (2S)-2-(1-Adamantylmethyloxycarbonylamino)-3-(4-(3-(4,5-dihydroimidazol-2-yl-carbamoyl)-propyloxy)phenyl)-propionsäure (5.2)

188 mg (0,32 mmol) (5.1) wurden in 10 ml Trifluoressigsäure/H₂O (95:5) gelöst. Nach 30 min wurde das Lösungsmittel im Vakuum entfernt und der Rückstand mit Diisopropylether digeriert. Nach Gefriertrocknung erhielt man 168 mg eines amorphen Pulvers von (5.2).

### Pharmakologische Untersuchungen

Die Hemmung der Knochenresorption durch die erfindungsgemäßen Verbindungen kann beispielsweise mit Hilfe eines Osteoclasten-Resorptions-Tests ("PIT ASSAY") beispielsweise analog WO 95/32710 bestimmt werden.

Die inhibitorische Wirkung der erfindungsgemäßen Verbindungen gegenüber dem Vitronectinrezeptor αᵥβ₃ kann z. B. wie nachfolgend beschrieben bestimmt werden.

Test zur Messung der Inhibierung der Bindung von 293 Zellen an humanes Vitronectin (bei den Testergebnissen mit Vn/293 Zelltest abgekürzt)

### 1. Reinigung von humanem Vitronectin

Humanes Vitronectin wird aus menschlichem Plasma isoliert und durch Affinitätschromatographie nach der Methode von Yatohyo et al., Cell Structure and Function, 1988, 23, 281-292 gereinigt.

### 2. Zelltest

293 Zellen, eine humane embryonale Nierenzellinie, die mit DNA-Sequenzen für die αᵥ und β₃ Untereinheiten des Vitronectinrezeptors αᵥβ₃ cotransfiziert sind, werden nach der FACS Methode unter dem Gesichtspunkt einer hohen Expressionsrate (> 500000 αᵥβ₃ Rezeptoren/Zelle) selektiert. Die ausgewählten Zellen werden kultiviert und erneut mittels FACS aussortiert, um eine stabile Zellinie (15 D) mit Expressionsraten >1000000 Kopien an αᵥβ₃ pro Zelle zu erhalten.

Eine Linbro 96well Gewebekulturplatte mit flachem Boden wird mit humanem Vitronectin (0,01 mg/ml, 0,05 ml/well) in Phosphat-gepufferter Kochsalzlösung (PBS) über Nacht bei 4°C belegt und anschließend mit 0,5 %igem BSA geblockt. Man stellt Lösungen der Testsubstanzen von 10⁻¹⁰-2x10⁻³ Mol/l in glucosehaltigem DMEM Medium her und gibt jeweils 0,05 ml/weil der Lösung auf die Platte. Die Zellen, die hohe Level an αᵥβ₃ exprimieren (z.B. 15 D) werden in glucosehaltigem DMEM Medium suspendiert und die Suspension wird auf einen Gehalt von 25000 Zellen/0,05 ml Medium eingestellt. 0,05 ml dieser Zellsuspension werden in jedes well gegeben und die Platte bei 37°C 90 min inkubiert. Die Platte wird 3 x mit warmem PBS gewaschen, um nichtgebundene Zellen zu entfernen. Die gebundenen Zellen werden in Zitratpuffer (25 mMol, pH 5,0), der 0,25 % Triton X-100 enthält, lysiert. Anschließend wird das Hexoseamidase-Substrat p-Nitrophenyl-N-acetyl-β-D-glucosaminid zugegeben und die Platte 90 min bei 37°C inkubiert. Die Reaktion wird mit einem Glycin (50 mMol)/EDTA (5 mMol)-Puffer (pH 10,4) gestoppt und die Absorption von jedem well bei 405-650 nm gemessen. Die Daten werden nach Standardverfahren ausgewertet.

Es wurden folgende Testergebnisse erhalten:

| | Vn/293 Zelltest IC₅₀ (µM) |
|---|---|
| Verbindung des Beispiels 1 | 0,032 |
| Verbindung des Beispiels 3 | 0,032 |

## Patentansprüche

1. Verbindung der Formel 1,
R¹-Y-A-B-D-E-F-G I,
worin bedeuten:
A -NH-C(O)-;
B (C₁-C₄)-Alkandiyl;
D -O-, -NR²-C(O)-, -C(O)-NR²- oder eine direkte Bindung;
E Phenylen oder Pyridindiyl;
F -CH₂-oder-C(O)NHCH₂-;
G 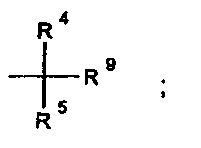
Y eine direkte Bindung;
R¹ H₂N-C(=NH)-, 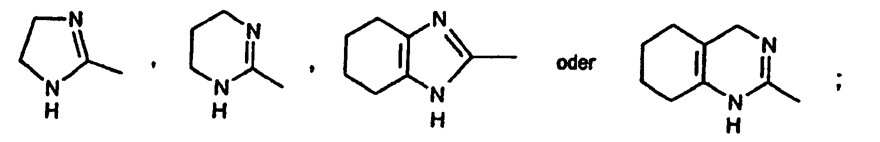
R² H oder (C₁-C₄)-Alkyl;
R⁴ R⁶OC(O)NH-;
R⁵ H;
R⁶ Adamantyl-1-(C₁-C₃)-alkylen, Adamantyl-2-(C₁-C₃)-alkylen, 1-Adamantyl,2-Adamantyl, wobei Adamantyl bevorzugt 1 oder 2fach durch (C₁-C₄)-Alkyl, Trifluormethyl, Phenyl, Benzyl, (C₁-C₄)-Alkoxy, Phenoxy oder Benzyloxy substituiert ist, oder (C₁₁-C₁₂)-Cycloalkyl, das wie vorstehend beschrieben 1- oder 2-fach substituiert sein kann;
R⁹ C(O)R¹⁰;
R¹⁰ OH, (C₁-C₆)-Alkoxy, Phenoxy, Benzyloxy oder (C₁-C₄)-Alkoxycarbonyloxy-(C₁-C₄)-alkandiyloxy;
in allen ihren stereoisomeren Formen und Gemische davon in allen Verhältnissen, und ihre physiologisch verträglichen Salze.

2. (2S)-2-(1-Adamantylmethyloxycarbonylamino)-3-(4-(3-guanidinocarbonyl-propyloxy)phenyl)-propionsäure

3. (2S)-2-((2-(1-Adamantyl)-ethyl)oxycarbonylamino)-3-(4-(3-guanidinocarbonyl-propyloxy)phenyl)-propionsäure

4. (2S)-2-(1-Adamantylmethyloxycarbonylamino)-3-(4-(2-(1,4,5,6-tetrahydropyrimidin-2-yl-carbamoyl)-ethyl) benzoylamino)-propionsäure

5. (2S)-2-(1-Adamantylmethyloxycarbonylamino)-3-(4-(3-(1,4,5,6-tetrahydropyrimidin-2-yl-carbamoyl)-propyloxy)phenyl)-propionsäure

6. (2S)-2-(1-Adamantylmethyloxycarbonylamino)-3-(4-(3-(4,5-dihydroimidazol-2-yl-carbamoyl)-propyloxy)phenyl)-propionsäure

7. Verbindung der Formel I gemäß einem oder mehreren der Ansprüche 1 bis 6 und/oder ihre physiologisch verträglichen Salze zur Verwendung als Arzneimittel.

8. Verbindung der Formel I gemäß einem oder mehreren der Ansprüche 1 bis 6 und/oder ihre physiologisch verträglichen Salze als Inhibitoren der Knochenresorption durch Osteoclasten, als Inhibitoren von Tumorwachstum und Tumormetastasierung, als Entzündungshemmer, zur Behandlung oder Prophylaxe von cardiovaskulären Erkrankungen, zur Behandlung oder Prophylaxe von Nephropathien und Retinopathien oder als Vitronectinrezeptor-Antagonisten zur Behandlung und Prophylaxe von Krankheiten, die auf der Wechselwirkung zwischen Vitronectinrezeptoren und deren Liganden bei Zell-Zell- oder Zell-Matrix-Interaktionsprozessen beruhen.

9. Pharmazeutische Zubereitung, enthaltend mindestens eine Verbindung der Formel gemäß einem oder mehreren der Ansprüche 1 bis 6 und/oder deren physiologisch verträgliche Salze neben pharmazeutisch einwandfreien Träger- und Zusatzstoffen.

## Claims

1. A compound of the formula I,
R¹-Y-A-B-D-E-F-G I,
in which:
A is -NH-C(O)-;
B is (C₁-C₄)-alkanediyl;
D is -O-, -NR²-C(O)-, -C(O)-NR²- or a direct bond;
E is phenylene or pyridinediyl;
F is -CH₂- or -C(O)NHCH₂- ;
G is 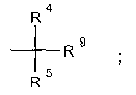
Y is a direct bond;
R¹ is H₂N-(-NH)-, 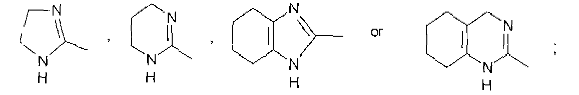
R² is H or (C₁-C₄)-alkyl;
R⁴ is R⁶OC(O)NH-;
R⁵ is H;
R⁶ is adamantyl-1-(C₁-C₃)-alkylene, adamantyl-2-(C₁-C₃)-alkylene, 1 adamantyl, 2-adamantyl, adamantyl preferably being substituted 1 or 2 times by (C₁-C₄)-alkyl, trifluoromethyl, phenyl, benzyl, (C₁-C₄)-alkoxy, phenoxy or benzyloxy, or (C₁₁-C₁₂)-cycloalkyl which can be substituted 1 or 2 times as above;
R⁹ is C(O)R¹⁰,
R¹⁰ is OH, (C₁-C₆)-alkoxy, phenoxy, benzyloxy or (C₁-C₄)-alkoxy-carbonyloxy-(C₁-C₄)-alkanediyloxy;
in all their stereoisomeric forms and mixtures thereof in any ratio; and their physiologically tolerable salts.

2. (2S)-2-(1-Adamantylmethyloxycarbonylamino)-3-(4-(3-guanidino-carbonyl-propyloxy)phenyl)-propionic acid.

3. (2S)-2-((2(1-Adamantyl)-ethyl)oxycarbonylamino)-3-(4-(3-guanidino-carbonyl-propyloxy)phenyl)-propionic acid.

4. (2S)-2-(1-Adamantylmethyloxycarbonylammo)-3-(4-(2-(1,4,5,6-tetrahydropyrimidin-2-yl-carbamoyl)-ethyl)benzoylamino)-propionic acid.

5. (2S)-2-(1-Adamantylmethyloxycarbonylamino)-3-(4-(3-(1,4,5,6-tetrahydropyrimidin-2-yl-carbamoyl)-propyluxy)-phenyl)-propionic acid.

6. (2S)-2-(1-Adamantylmethyloxycarbonylamino)-3-(4-(3-(4,5-dihydroimidazol-2-yl-carbamoyl)-propyloxy)-phenyl)-propionic acid.

7. A compound of the formula I as claimed in one or more of claims 1 to 6 and/or a physiologically tolerable salt thereof for use as medicaments.

8. A compound of the formula I as claimed in one or more of claims 1 to 6 and/or a physiologically tolerable salt thereof as inhibitors of bone resorption by osteoclasts, as inhibitors of tumor growth and tumor metastasis, as antiinflammatories, for the treatment or prophylaxis of cardiovascular disorders, for the treatment or prophylaxis of nephropathies and retinopathies or as vitronectin receptor antagonists for the treatment and prophylaxis of diseases which are based on the interaction between vitronectin receptors and their ligands in cell-cell or cell-matrix interaction processes.

9. A pharmaceutical preparation comprising at least one compound of the formula I as claimed in one or more of claims 1 to 6 and/or its physiologically tolerable salts in addition to pharmaceutically innocuous excipients and additives.

## Revendications

1. Composé de formule I,
R¹-Y-A-B-D-E-F-G I,
dans laquelle:
A signifie -NH-C(O)- ;
B signifie un alcanediyle (en C₁-C₄);
D signifie -O-, -NR²-C(O)-, -C(O)-NR²- ou une liaison directe ;
E signifie un phénylène ou un pyridindiyle ;
F signifie -CH₂- ou -C(O)NHCH₂-
G signifie 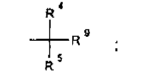
Y signifie une liaison directe;
R¹ signifie -H₂N-C(=NH)-: 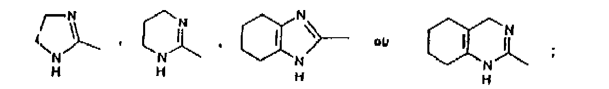
R² signifie 11 ou un alkyle en (C₁-C₄) ;
R⁴ signifie R⁶OC(O)NH-;
R⁵ signifie H ;
R⁶ signifie adamantyl-1-alkylène (en C₁-C₃), adamantyl-2-alkylène (en C₁-C₃), 1-adamantyle, 2-adamantyle, dans lequel l'adamantyle est de préférence mono ou disubstitué par un groupe alkyle en C₁-C₄, trifluorométhyle, phényle, benzyle, alcoxy en C₁-C₄, phénoxy ou benzyloxy ; ou R⁶ est un cycloalkyle en C₁₁-C₁₂ qui peut être mono ou disubstitué, comme décrit précédemment.
R⁹ signifie C(O)R¹⁰;
R¹⁰ signifie OH, alcoxy (en C₁-C₆), phénoxy, benzyloxy ou alcoxycarbonyloxy (en C₁-C₄)-alcandiyloxy (en C₁-C₄);
sous toutes ses formes stéréoisomériques et leurs mélanges en toutes proportions, et ses sels physiologiquement acceptables.

2. Acide (2S)-2-(1-adamantylmethyloxycarbonylamino)-3-(4-(3-guani-dinocarbonyl-propyloxy)phényl)-propionique.

3. Acide (2S)-2-((2-(1 -adamantyl)éthyl)oxycarbonylamino)-3-(4-(3-guanicinocarbonyl-propyloxy)phenyl)-propionique.

4. Acide (2S)-2-(1-adamantylméthyloxycarbonylamino)-3-(4-(2-(1,4,5,6-tétrahydropyrimidin-2-yl-carbonyl)éthyl)benzoylamino)-propionique.

5. Acide (2S)-2-(1-adamantylméthyloxycarbonylamino)-3-(4-(3-(1,4,5,6-tétrahydropyrimidin-2-yl-carbonyl)-propyloxy)phényl)-propionique.

6. Acide (2S)-2-(1 -adamantylméthyloxycarbonylamino)-3-(4-(3-(4,5-dihydroimidazole-2-yl-carbonyl)-propyloxy)phenyl)-propionique.

7. Composé de formule 1 selon l'une ou plusieurs des revendications 1 à 6, et/ou leurs sels physiologiquement acceptables pour servir de médicament.

8. Composé de formule 1 selon l'une ou plusieurs des revendications 1 à 6, et/ou leurs sels physiologiquement acceptables en tant qu'inhibiteurs de la resorption osseuse par ostéoclaste, en tant qu'inhibiteur de la croissance tumorale et de métastase tumorale, en tant qu'anti-inflammatoire, en tant que médicament pour le traitement ou la prophylaxie de maladies cardiovasculaires, en tant que traitement ou prophylaxie de néphropathie et de rétinopathie ou en tant qu'antagoniste de récepteur de la vitronectine pour le traitement et la prophylaxie de maladies qui reposent sur l'interaction entre le récepteur de victronectine et leurs ligands par des processus d'interaction cellule-cellule ou cellule-matrice.

9. Composition pharmaceutique, contenant au moins un composé de formule 1 selon l'une ou plusieurs des revendications 1 à 6 et/ou leurs sels physiologiquement acceptables et un excipient ou un additif pharmaceutiquement acceptable.
